# EUROPEAN PATENT APPLICATION

(11) **EP 1 154 016 A2**
(43) Date of publication of application: **14.11.2001**
(21) Application number: 01110918.8
(22) Date of filing: 05.05.2001
(51) Int. Cl.: C12N 5/06, C07K 16/18, G01N 33/53, A61B 8/00

(54) **Methods of isolating trophoblast cells from maternal blood**

(30) Priority: 12.05.2000 US 569475
(71) Applicant: Roche Diagnostics GmbH, 68305 Mannheim (DE); F. HOFFMANN-LA ROCHE AG, 4070 Basel (CH)
(72) Inventor: Mahoney, Walter, Benicia, California 94510 (US); Schueler, Paula, Benicia, California 94510 (US); Yamanishi, Douglas, Moraga, California 94566 (US)

(57) **Abstract**

Methods for isolating trophoblast cells from pregnant women are provided. A central maternal blood sample is obtained, typically from the uterine wall of a pregnant woman. The trophoblast cells are isolated from the central maternal blood sample.

## Description

### TECHNICAL FIELD

The invention relates generally to the field of prenatal diagnostics, more particularly to the field isolating fetal cells from maternal samples.

### BACKGROUND ART

Fetal cells have long been known to escape into the maternal circulation. These cells have been viewed as an attractive source of fetal cells for genetic analysis of the developing fetus. Obtaining a blood sample from a peripheral blood vessel, typically a superficial vein in the lower arm or hand, is relatively non-invasive and poses virtually no risk to the mother or the developing fetus, features lacking in current methods of obtaining fetal cells (amniocentesis and chorionic villus sampling). In addition, since fetal cells peak in the maternal circulation at about 10-16 weeks of gestation, it is possible to perform the genetic analysis at an early stage in pregnancy. However, fetal cells are extremely rare in peripheral maternal blood, on the order of 1 to 50 cells per 10⁷ nucleated blood cells. These low levels of fetal cells make even minimal levels of non-specific binding problematic for affinity separation of fetal cells from maternal cells.

A number of fetal cells are known to make their way into the maternal circulation, including leukocytes, trophoblast cells and nucleated red blood cells. Trophoblasts are large cells derived from the placenta, and have no counterpart in nonpregnant adults. Thus, identifying trophoblast-specific or associated markers is viewed as a substantially easier task than identifying markers for fetal leukocytes or erythroid cells. A number of antibodies specific for trophoblast cells have been identified, and have been used to detect trophoblast cells in peripheral blood samples. See, for example, U.S. Patent No. 5,503,981 Johnson et al. (1981, *Am. J. Reprod. Immunol.* **1**(2):83-87 ), Sunderland et al. (1981, *Immunology* **43**(3):541-546.), Lipinski et al. (1981, *Proc. Natl. Acad. Sci. USA* **78**(8):5147-5150), Loke and Day (1984, *Am. J Reprod. Immunol.* **5**(3):106-108), and Anderson et al. (1987, *J. Reprod. Immunol.* **10**(3):231-257).

A number of different methods have been proposed for isolating trophoblast cells can be isolated from maternal blood samples. However, the scarcity of trophoblasts in maternal peripheral blood samples makes isolating pure preparations of trophoblast cells extremely difficult, as even low levels of carryover or crossreaction results in a significant decrease in purity of the preparation.

Accordingly, there is a need in the art for methods of obtaining maternal blood samples containing large numbers of fetal cells.

### SUMMARY OF THE INVENTION

The invention provides methods of isolating trophoblast cells from a pregnant female by obtaining a central maternal blood sample from the pregnant female and isolating trophoblast cells from the central maternal blood sample using at least one affinity reagent which binds to a trophoblast marker.

In certain embodiments, the central maternal blood sample is obtained from the uterine wall of the pregnant female. Such blood samples may be obtained by inserting a needle into the face of said individual's cervix or by inserting a needle through said individual's uterine os and into said individual's uterine wall. In other embodiments, the central maternal blood sample is obtained by venipuncture of a vessel draining the uterus.

In certain embodiments, the central maternal blood sample is preferably obtained at from about 6 to about 16 weeks of gestation.

In certain embodiments, the central maternal blood sample is obtained using a needle that is at least 20 gauge. Additional embodiments utilize a needle that is at least 18 gauge.

In certain embodiments, the trophoblast cells are isolated using an affinity reagent that binds a trophoblast specific marker. Alternate embodiments utilize an affinity reagent that binds a trophoblast associated marker to isolate the trophoblast cells.

In certain embodiments, a single affinity reagent is used to isolate the trophoblast cells, but the use of multiple (*e.g*., at least two) affinity reagents is also contemplated. In certain embodiments the affinity reagent is an antibody, and may be a monoclonal antibody.

### DETAILED DISCLOSURE OF THE INVENTION

We have discovered new methods of isolating conceptus cells from maternal blood. The methods of the invention find use in any field which requires nucleated cells from a developing fetus or embryo, such as prenatal genetic testing. The instant methods are advantageous compared to amniocentesis and chorionic villus sampling because the instant methods are less invasive and pose little, if any, risk to the developing fetus or embryo.

The invention utilizes maternal blood samples rich in trophoblast cells. A maternal blood sample rich in trophoblasts is obtained from maternal central, rather than peripheral, blood, and trophoblasts are isolated therefrom. Samples of central maternal blood are most conveniently obtained from the uterus, preferably the wall of the uterus and endometrium. Trophoblast cells are isolated from the central maternal blood sample by the use reagents specific for or associated with trophoblast cells.

### Definitions

Pregnancy can be staged reference to "weeks of pregnancy" or "weeks of gestation". Those in the field of obstetrics generally use "weeks of pregnancy", which is calculated from the date at which the mother's most recent menses began, with a 40 week term of pregnancy considered normal. Weeks of gestation (alternately "gestational age") are calculated relative to the date of conception. Because conception is rarely directly documented (except in the case of *in vitro* fertilization), the date of conception is typically calculated from other indicators of gestational age, such as crown-rump (CR) length. Because ovulation takes place at some point after the beginning of menses, generally about two weeks after the beginning of menses, gestational age (in weeks) is always less than the weeks of pregnancy.

An "embryonic cell" is a conceptus cell which is isolated from an embryonic stage of development. The embryonic period begins at fertilization, and is considered to end when the major organ systems have been formed. In humans, the embryonic period is considered to end at the completion of 10^{th} week of gestation.

A "fetal" cell is a conceptus cell which is isolated from a fetal stage of development. The fetal period of development begins at the end of the embryonic period of development, and continues until birth. In humans, the fetal period is generally considered to begin at the end of 10 weeks of gestation.

The term "conceptus cell", as used herein, refers to a cell or cell fragment which is derived from mammalian products of conception. Conceptus cells may be derived from the embryonic or fetal stages of development, and may be derived from the embryonic or fetal corpus or from associated tissues such as embryonic/fetal membranes or the embryonic/fetal portion of the placenta.

The term "trophoblast cell", as used herein, refers to a conceptus cell or cell fragment which is derived from the trophoblast portion of the embryonic/fetal portion of the placenta. Trophoblast cells include syncytiotrophoblasts (villous and non-villous), including nucleated fragments thereof, and cytotrophoblasts.

A "trophoblast specific marker" is a marker *(e.g.,* protein or other material found in or on a trophoblast cell) which is not found on any other cell type in the organism. A "trophoblast associated marker" is a marker which is found on one or more additional cell types in the organism.

An "erythroid cell" is an immature cell of the erythroid lineage (*i.e*., a cell of the erythroid lineage which is not a mature erythrocyte). Erythroid cells include reticulocytes, orthochromatic erythroblasts, polychromatophilic erythroblasts, basophilic erythroblasts, proerythroblasts, colony forming unit-erythroid (CFU-E) and burst forming unit-erythroid (BFU-E).

The term "individual", as used herein, refers to a mammal, such as a pig, cow, horse, dog, cat, or a primate. Preferably, a primate individual is a human individual.

The term "central circulation" as used herein, refers to circulation within the trunk of a mammalian individual (*i.e*., circulation within the thorax, abdomen or pelvis). The term "central circulation" excludes peripheral circulation (*i.e*., circulation within the appendages). Likewise, a "central blood sample" is a blood sample that is obtained from the central circulation (*i.e*., is not obtained from the peripheral circulation).

By "binding effective amount of reagent", as used herein, is meant an amount of an affinity sufficient to bind to the target cells and be used in the isolation of such cells. The affinity reagent may be any molecule which binds specifically to a trophoblast specific or associated marker, although antibodies are preferred, and monoclonal antibodies are particularly preferred.

The term "comprising" and its cognates, as used herein, is used in the inclusive sense (*i.e*., synonymous with the word "including" and its cognates).

The instant invention provides methods of isolating trophoblast cells from maternal blood. Trophoblast cells isolated by the methods of the invention are useful in any technique requiring fetal cells, such as prenatal genetic testing. Current standard of practice in the U.S. is to perform prenatal genetic testing in high risk pregnancies (*e.g*., maternal age of ≥35, family history of genetic disorders such as cystic fibrosis, Huntington's chorea, and the like).

While maternal blood samples have long been viewed as an attractive source of conceptus cells for prenatal genetic testing, previous efforts to isolate conceptus cells from maternal blood have utilized peripheral blood samples. The instant invention utilizes central maternal blood samples as a source for conceptus cells, particularly trophoblast cells.

Maternal central blood samples may be obtained at any time between implantation (*e.g*., about 1-2 weeks gestational age, or about 3-4 weeks of pregnancy) and birth, although samples are preferably obtained not later than about 26 weeks of pregnancy. More preferably, the maternal central blood sample is obtained at about 8 to 18 weeks of gestation, more preferably about 10 to 16 weeks of gestation.

Central maternal blood samples are obtained from maternal central circulation (*e.g*., from the venous circulation of the trunk of the mother, such as from the internal iliac vein, the common iliac vein, or the inferior vena cava), and are preferably obtained from the uterus, more preferably the uterine wall. The exact manner of obtaining the central maternal blood sample will depend on the desired site for collection of the sample, as will be recognized by one of skill in the art.

Blood samples from the internal iliac vein, the common iliac vein, or the inferior vena cava are preferably obtained by percutaneous insertion of a needle into the vein, although a catheter may also be introduced and advanced to a desired collection point. When a needle is used to directly obtain a blood sample, the target blood vessel is preferably localized using an appropriate technology, such as ltrasonography, preferably Doppler ultrasonography. When a catheter is used to obtain the sample, the orifice of the catheter is preferably advanced to a point at which it can collect venous blood draining from the uterus. For example, a catheter may be introduced into the femoral vein and advanced into the common iliac vein or near the point at which the common iliac vein joins with the inferior vena cava.

It is preferred that the central maternal blood sample be obtained from the uterine wall. This approach has the advantage of being relatively noninvasive. The woman is placed in the normal position for a pelvic gynecologic exam (*i.e.*, supine, knees and hips flexed, and feet and knees spread), and the "face" of the cervix (the surface of the cervix which projects into the vagina) is visualized. Preferably, the face of the cervix is visualized by dilation of the vaginal canal, preferably by insertion of a speculum (preferably a speculum warmed to near, but not above body temperature, *e.g*., about 30° to 37° C for a human) into the vaginal canal, and gentle opening of the speculum. A large gauge needle (*i.e*., larger than about 20 gauge), preferably at least 18 gauge, is inserted into the wall of the uterus, either by insertion through the face of the cervix or by passing the point of the needle through the uterine os into the intrauterine space, then inserting the needle into the uterine wall. The needle is not inserted into the placenta. If the maternal central blood sample is obtained by passing the needle through the os of the cervix, it is preferred that the position of the developing embryo or fetus be determined by ultrasound exam prior to obtaining the sample, to insure that insertion of the needle will not endanger the developing embryo or fetus or placenta, and to guide needle placement within the uterine wall.

The needle may be attached to any device useful for collecting blood, such as a syringe, a flexible container *(e.g.,* a "blood bag") or a low pressure blood collecting apparatus (*e.g*., a glass vial with a resilient stopper sealing the vial and preserving a partial vacuum contained therein, such as a VACUTAINER® blood collection tube (Becton, Dickinson and Company). Preferably, the collecting device contains an anticoagulant, such as citrate or a salt of heparin, to permit processing of the sample without clot formation.

The volume of the central maternal blood sample obtained will depend on the preferences of the practitioner, the requirements of the isolation technology to be used on the sample, and the intended use of the isolated trophoblasts (*e.g*., the requirements of the assay to be preformed on the isolated trophoblast cells). Preferably, at least about one mL of blood is obtained, more preferably at least about two mL of blood are obtained. When the central maternal blood sample is obtained directly from the uterine wall, the blood sample is preferably about 2 to about 10 mL.

The central maternal blood sample may be processed to reduce maternal cells having very different properties from trophoblast cells (*e.g*., erythrocytes) using any convenient technology, such as filtration or density gradient centrifugation. However, preliminary processing is preferably avoided, due to the inevitable loss of trophoblast cells associated with such processing.

The central maternal blood sample is contacted with a binding-effective amount of an affinity reagent specific for a marker specific for or associated with trophoblast cells for a time and under conditions sufficient for said antibody to bind to trophoblast cells. Any affinity reagent specific for a trophoblast-specific or trophoblast-associated marker may be used in the method of the invention, although it is preferred that when the reagent is specific for a trophoblast associated marker, the trophoblast associated marker is one which is not found on maternal blood cells. More preferably, the affinity reagent is specific for a trophoblast specific marker.

It should be recognized that trophoblasts may be isolated using a single affinity reagent or a combination of two or more affinity reagents. In certain embodiments, the combination of affinity reagents comprises at least one affinity reagent specific for a trophoblast-specific and at least one affinity reagent specific for a trophoblast-associated marker.

The affinity reagent is preferably a antibody, more preferably a monoclonal antibody. A number of trophoblast specific antibodies are known in the art, such as the antibodies H315, disclosed in Johnson et al. (1981, *Am. J. Reprod. Immunol.* **1**(2):83-87 ), NDOG1, disclosed in Sunderland et al. (1981, *Immunology* **43**(3):541-546.), Trop. 1 and Trop. 2, disclosed in Lipinski et al. (1981, *Proc. Natl. Acad. Sci. USA* **78**(8):5147-5150), 18B/A5, disclosed in Loke and Day (1984, *Am. J Reprod. Immunol.* **5**(3):106-108), anti-trophoblast antibodies are also discussed in Anderson et al. (1987, *J. Reprod. Immunol.* **10**(3):231-257). Monoclonal antibodies FDO161G, FDO66Q and FDO338P are disclosed in U.S. Patent No. 5,503,981. Additional monoclonal antibodies, such as LK26 (specific to a human choriocarcinoma antigen, Signet), 5T4 (specific to a villous syncytiotrophoblast antigen, Pharmingen) are available commercially.

Alternately, monoclonal antibodies specific for trophoblast markers can be isolated *de novo,* using currently available monoclonal antibody production technology and trophoblasts isolated from placentas. Preferably the placentas used for monoclonal antibody production are from first trimester or early second trimester pregnancies (*e.g.,* from about 6 to 16 weeks of pregnancy), corresponding approximately to the timepoints at which trophoblast cells are isolated from maternal blood in accordance with the invention. Preferably, trophoblast cells are isolated from placentas obtained from elective terminations of apparently healthy pregnancies performed by aspiration. Associated clotted blood and any adherent decidua are carefully dissected from the placentas, then trophoblast cells are isolated. Syncytiotrophoblast may be isolated by gently teasing the placentas through a fine (*e.g*., 250-mesh) sieve. The sheets of syncytiotrophoblast, being significantly larger than contaminating cells, readily sediment at unit gravity in a physiologically acceptable salt solution such as Earle's or Hank's balanced salt solution. The material passing through the mesh is allowed to sediment, the supernatant, included suspended cells, is discarded, and the sedimented cells are washed several times by resuspension and sedimentation. Isolated trophoblast cells may be used directly, cultured to expand cell number, or extracted to produce a cell-free antigen preparation. If the cells are cultured, successful isolation and culture of trophoblasts may be assessed by assay of chorionic gonadotropin production in the culture.

Trophoblast antigen preparations may be produced using standard biochemical techniques. Because it is preferable to isolate trophoblasts without first fixing and permeabilizing the central maternal blood sample, surface trophoblast markers are preferred. Accordingly, membrane preparations and solubilized membrane preparations from trophoblast cells are preferred trophoblast cell antigen preparations. Methods of producing membrane preparations and solubilized membrane preparations are well known in the art and nee d not be discussed here.

The trophoblast suspension or a trophoblast antigen preparation may be used for immunization of an animal, preferably a rodent, more preferably a mouse, for antibody production. Preferably, mice suited for monoclonal antibody production (*e.g*., Balb/C mice) are immunized with the trophoblast cell suspension or trophoblast antigen preparation, preferably by repeated intraperitoneal (IP) injection.

Alternately, antibodies may be isolated using scFv screening technology, wherein scFv antibodies are selected by exposing a library of scFv antibodies displayed on the surface of phage to trophoblast cells or trophoblast cell antigens, and isolating those phages which bind to the cells or antigen preparation. Preferably, a naive human library is used, such as the Griffiths library (Griffiths et al., 1993, *EMBO J.* **12**(2):725-734).

The trophoblast-specific affinity reagent is used to isolate trophoblast cells away from maternal cells present in the central maternal blood sample. Isolation may be accomplished by a variety of techniques well known in the art, including cell sorting, especially fluorescence-activated cell sorting (FACS), by using an affinity reagent bound to a substrate (*e.g*., a plastic surface, as in panning), or by using an affinity reagent bound to a solid phase particle which can be isolated on the basis of the properties of the beads (*e.g*., colored latex beads or magnetic particles). As will be apparent to one of skill in the art, the trophoblast-specific affinity reagent may be bound directly or indirectly (*e.g*., via a secondary antibody) to the dye, substrate, or particle.

For isolation of trophoblast cells by cell sorting, the affinity reagent is labeled directly or indirectly with substance which can be detected by a cell sorter, preferably a dye. Preferably, the dye is a fluorescent dye. A large number of different dyes are known in the art, including fluorescein, rhodamine, Texas red, phycoerythrin, and the like. Any detectable substance which has the appropriate characteristics for the cell sorter may be used (*e.g*., in the case of a fluorescent dye, a dye which can be excited by the sorter's light source, and an emission spectra which can be detected by the cell sorter's detectors).

For isolation of trophoblast cells using solid-phase particles, any particle with the desired properties may be utilized. For example, large particles *(e.g.,* greater than about 90-100 µm in diameter) may be used to facilitate sedimentation. Preferably, the particles are "magnetic particles" (*i.e*., particles which can be collected using a magnetic field). Magnetic particles are now commonly available from a variety of manufacturers.

When a dye or a solid phase particle is used in conjunction with the affinity reagent to isolate trophoblast cells from the central maternal blood sample, the dye or solid phase particle is used to isolate the trophoblast cells, the dye or solid phase particle may be directly or indirectly linked to the affinity reagent. Whether the affinity reagent is directly or indirectly linked is left to the discretion of the practitioner. Directly labeled affinity reagents are produced by linking the dye or solid phase particle to the affinity reagent by, for example, covalent linkage of a dye or by adsorption to a solid phase particle. Affinity reagents may be indirectly labeled using a variety of methods known in the art, such as using a "secondary antibody" (a directly labeled antibody which binds specifically to the affinity reagent), or by exploiting a binding pair such as biotin and streptavidin (*e.g*., by derivatizing the affinity reagent with biotin, and using directly labeled streptavidin to bind label to the affinity reagent).

For isolation of trophoblast cells using an affinity reagent bound to a substrate, the affinity reagent is preferably adsorbed or bound directly to the substrate. Preferably, the substrate is the surface of a plastic plate or flask, and the affinity reagent is directly adsorbed to the surface. Adsorption is easily accomplished for most affinity reagents, and when the affinity reagent is an antibody, adsorption is accomplished by simply incubating a solution containing the antibody on the substrate. Alternately, a modified substrate may be used, such as a substrate modified with avidin or streptavidin, and an affinity reagent modified with biotin, or an amine-derivatized substrate activated with a bifunctional crosslinking agent. Preferably, the affinity reagent is adsorbed to the substrate by incubating a solution containing the affinity reagent on the substrate.

After isolation using the affinity reagent, the isolated trophoblast cells may be used directly for perinatal genetic testing, or they may be cultured to expand cells numbers and to facilitate karyotypic analysis.

Perinatal genetic testing is preferably carried out using *in situ* hybridization (ISH) methods, more preferably fluorescence *in situ* hybridization, although amplification-based (*e*.*g*., PCR-based) methods are also useful.

Methods for *in situ* hybridization (ISH) are well known in the art. ISH is normally carried out on fixed, permeabilized trophoblast cells which have been fixed to an insoluble substrate, such as a poly-L-lysine-coated glass slide or polystyrene plate or dish, although ISH may also be carried out on fixed cells in suspension. Where the cells are adhered to a substrate, the substrate is preferably transparent to visible and ultraviolet light (*e*.*g*., glass), to allow for use of fluorescent dyes as labels. As will be appreciated by one of skill in the art, materials and solutions used preparation of cells for ISH and for ISH itself are preferably RNase-free.

Generally, a suspension of cells is made in a solution comprising little or no added protein (*e.g*., serum free medium or a balanced salt solution) and placed on substrate which has been derivatized to allow attachment of cells by use of a crosslinking agent. Preferably, the substrate is modified by coating with poly-L-lysine or by "subbing" with gelatin. The cell suspension is placed on the substrate, generally as a small "pool" or drop on the surface of the substrate, and the cells are allowed to attach to the substrate by settling under normal gravity for a period of time, preferably at least about 10, 20 or 30 minutes, although the cells may be "spun" onto the substrate by the use of a centrifuge with an appropriate rotor adapted to hold the substrate. Attachment of the cells onto the substrate is preferably accomplished under conditions of humidity approaching 100%, as will be apparent to one of skill in the art.

After the cells have attached to the substrate, the cells are crosslinked to the substrate (or to the derivative bound to the substrate) using a fixative. Any appropriate fixative may be used, including acid alcohol solutions, acid acetone solutions, aldehyde fixatives, homobifunctional crosslinking agents such as N-hydroxysuccinimide (NHS) esters (*e.g*., disuccinimidyl suberate, disuccinimidyl glutarate, and the like) and heterobifunctional crosslinking agents known in the art. Preferably, an aldehyde fixative such as formaldehyde, paraformaldehyde or glutaraldehyde, is used to crosslink cells to poly-L-lysine or gelatin coated substrates. Preferably, the cells are fixed to the substrate by placing the substrate with attached cells into a bath of fixative solution, although fixation may be accomplished by replacing the pool or drop of liquid containing the cells with a similar volume of fixative. The attached cells and substrate are incubated in the fixative for a period of time appropriate to the particular fixative selected by the practitioner, preferably about 20 minutes in the case of 4% paraformaldehyde.

After fixation, the substrate may be rinsed, typically with a buffered saline solution such as phosphate buffered saline or tris-buffered saline, dehydrated using a series of ethanol baths (*e.g*., by incubating the fixed cells in 50%, 70%, 95%, and 100% ethanol for 2-5 minutes each) air dried, and stored for later ISH processing. Where the cell/substrate preparation is intended for immediate ISH processing, the cells must still be permeabilized, preferably by incubating the cell/substrate preparation in 50% ethanol, although detergent solutions, such as 0.01 to 0.1% t-octylphenoxypolyethoxyethanol or polyoxyethylenesorbitan monolaurate, may also be used.

Alternately, the cells may be fixed in solution using an appropriate fixative, rinsed, dehydrated and embedded in paraffin, then sectioned and adhered to glass slides using conventional histologic processing techniques. Prior to processing for ISH, cells processed in this matter must be de-paraffinized, typically by use of a xylene bath, and rehydrated by processing through progressively less concentrated ethanol solutions, as is well known in the art.

The cells to be analyzed are first denatured, generally by use of extreme pH (*e.g*., 0.2 N HCl for 10-30 minutes at room temperature) followed by high temperature (*e.g*., 10-20 minutes at 70° C in 2 x SSC), and an additional digestion with a non-specific protease (*e.g*., pronase) may be included as well. After denaturation, a post-fixation step is preferably performed by incubating the denatured cells in fixative (*e.g*., five minutes in 4% paraformaldehyde at room temperature), followed by rinsing in a buffered salt solution.

Non-specific binding sites on the cell/substrate preparation are preferably blocked prior to hybridization, typically by acetylation and modification of free sulfur groups. Preferably such blocking is carried out by incubating the cell/substrate preparation in a sulfur reducing agent (*e.g*., 10 mM dithiothreitol, DTT, in buffered saline at elevated temperature, such as 10 minutes at 45° C), followed by incubation with DTT, iodoacetamide, and N-ethylmaleimide (*e.g*., 10 mM DTT, 10 mM iodoacetamide, 10 mM N-ethylmaleimide for 30 minutes at 45° C). Additional blocking of polar and charged groups may be accomplished by incubation of the cell/substrate preparation in acetic anhydride (*e.g.*, 0.25 to .5% for 5-10 minutes at room temperature).

The probe is denatured prior to hybridization with the prepared cells. Normally, the probe is precipitated in ethanol, then redissolved in a small volume of solvent such as 2 x SSC, 1 x TEA, or formamide, heat denatured by incubating at 70° C or higher for 10-20 minutes, then added to a hybridization mixture. A non-specific, unlabeled DNA, such as sonicated salmon sperm DNA is preferably denatured along with the probe. Generally, when more than one probe is used, the probes are hybridized with the cells at the same time, although use of multiple probes does require use of divergent labeling systems to avoid signal crossover.

Hybridization is typically carried out at elevated temperature in hybridization mix containing a buffered salt solution (*e.g*., *4* x SSC), a high molecular weight polymer to increase the effective concentration of the probe(s) (*e.g*., 20% dextran sulfate), and a protein blocking agent (*e.g*., 2 mg/mL high purity bovine serum albumin). Hybridization is typically carried out under a coverslip which may be anchored in place with rubber cement or any other material which serves to temporarily anchor the coverslip and reduce evaporation of the hybridization mixture. Hybridization is preferably carried out under conditions where the hybridization temperature is 12-20° C below the melting temperature (Tₘ) of the probe. The Tₘ of a long polynucleotide can be found as Tₘ = 81.5 - 16.6(log₁₀[Na⁺]) + 0.41(%G + C) - 0.63(%formamide) - 600/N, where N = the length of the selectively hybridizable polynucleotide under study, while the Tₘ of oligonucleotides from about 70 to 15 nucleotides in length may be found as Tₘ = 81.5 - 16.6(log₁₀[Na+]) + 0.41(%G + C) - 600/N, and the Tₘ of short oligonucleotides of ≤14 nucleotides may be found as Tₘ = 2(A+T) + 4(G+C), where A, T, G and C are the numbers of adenosine, thymidine, guanosine and cytosine residues, respectively. Hybridization may be accomplished in as short a period as 2-4 hours, although longer hybridization incubations are also acceptable. Alternately, glycerol-based ISH technology, such as that disclosed in International Patent Application No. WO 96/31626 or U.S. Patent No. 5,948,617, may be used.

After the hybridization incubation is completed, the hybridization solution is removed, and the cells are washed, typically for 15 minutes each in 50% formamide/2 x SSC at 37° C, 2 x SSC at 37° C, and 1 x SSC at room temperature. After washing is completed, the cells are incubated the detection reagent (*e.g.*, fluorescently-labeled avidin or streptavidin for a biotinylated probe). The exact conditions of the incubation with the detection reagent will vary depending on the exact identity of the detection reagent, but is typically accomplished by incubation for 30-60 minutes at 37° C in a chamber protected from ambient light (to reduce photobleaching of the fluorescent label), although signal amplification techniques generally require multiple incubations, as will be apparent to one of skill in the art. Amplification techniques such as the use of secondary antibodies which bind to a primary detection reagent or enzymatic amplification may be employed if so desired. Excess detection or amplification reagent is washed away, typically by rinsing with a buffered salt solution (*e.g*., 4 x SSC) at room temperature. Optionally, a rinse including a detergent (*e.g*., 0.1% t-octylphenoxypolyethoxyethanol) in the buffered salt solution may be incorporated in the wash protocol.

Genomic DNA in the cells may be counterstained by incubation with a double-stranded DNA-binding dye, such as propidium iodide or 4,6-diamidino-2-phenylindole (DAPI) and rinsing away unbound dye.

Where trophoblast cells are processed as cells in suspension, the cells are carried through an substantially similar process, except that the cells collected by centrifugation or filtration after each step (*e.g*., after fixation, each wash step, etc.).

After hybridization, labeling with detection reagent and counterstaining, the cells are preferably sealed under a coverslip with an anti-fading reagent appropriate to the fluorescent dye(s) used in the detection reagent. The appropriate anti-fading reagent can be easily selected by the skilled practitioner.

Fetal cells may be detected by the use of any convenient fluorescent microscopy technique, including epifluorescence microscopy, confocal fluorescence microscopy, and other techniques known in the art. Results of microscopy may be stored on photographic negatives, photographic plates, or on magnetic or optical storage media when a CCD camera or other electronic imaging equipment is used. Alternately, cells which are processed as cells in suspension may be analyzed using FACS technology.

A wide variety of diagnostic assay technologies and probes are available for detection of chromosomal abnormalities and/or genetic diseases. For example, U.S. Patent No. 5,447,841 discloses probes specific for chromosome 21, which may be utilized in a diagnostic assay for trisomy 21 (*i.e*., Down's syndrome). Multiple genetic disorders may be assayed in a single test utilizing the multiplex FISH methods disclosed in U.S. Patent No. 6,007,994.

Amplification methods are generally more easily conducted than ISH-based methods, although quantitation can be difficult, so amplification-based assays are generally used for determination of the presence of a particular mutation or allele, rather than detection of a trisomy. Generally, DNA is extracted from the isolated trophoblast cells, and used as a template for an amplification reaction. The amplification reaction utilizes a pair of primers, a DNA polymerase, and mononucleotides. The exact primers used will depend on the particular genetic mutation or allele that is the target of the assay. The presence of a particular genetic mutation or allele is typically signaled by production of a amplification product of a predetermined size. In some amplification methods, "nested" amplification is used, whereby an iterative amplification process is utilized; a fragment of the product of a first amplification reaction is amplified using a second pair of primers. Nested amplification is generally considered more accurate, although it does add several additional steps to the assay.

Karyotyping is well known in the art. Karyotyping analysis is generally performed on cells which have been arrested during mitosis by the addition of a mitotic spindle inhibitor. Accordingly, it is preferred that the trophoblast cells be cultured prior to karyotyping. Methods for culture of trophoblast cells are known in the art. Preferably, the trophoblast cells are placed in a culture medium which has been supplemented with growth factors, such as conditioned medium from human decidua or granulosa cells. A mitotic spindle inhibitor such as colchicine is added to the culture to block those cells undergoing mitosis, and the cells are prepared for karyotypic analysis. Preferably, a Giemsa-stained chromosome spread is prepared, allowing analysis of chromosome number as well as detection of chromosomal translocations.

### EXAMPLES

### Example 1: Isolation of trophoblast cells from a central maternal blood sample

Trophoblast cells were isolated from central maternal blood samples of pregnant women undergoing chorionic villus sampling (CVS). Central maternal blood samples were obtained by dilating the vagina with a speculum, then withdrawing blood from the uterine wall using an 18 gauge spinal needle attached to a heparinized syringe. Approximately 3-10 milliliters (mL) were obtained from each woman. Matching CVS samples were also obtained from each woman. Samples were obtained from women carrying fetuses of 16 (37626), 12 (37636) and 13 (37637) weeks.

Magnetic beads coated with anti-mouse IgG antibodies bound via a DNA linker (Dyanbeads® Pan Mouse IgG, Dynal AS) were incubated with antibodies LK26 (specific to a human choriocarcinoma antigen, Signet), 5T4 (specific to a villous syncytiotrophoblast antigen, Pharmingen), 111.6 (specific to the extracellular domain of epidermal growth factor receptor, Neo Markers), 380 (specific to plasminogen activator inhibitor I, American Diagnostics), and N12 (specific to c-erbB-2/HER-2, NeoMarkers) to produce a magnetic affinity reagent. The blood samples were diluted with PB (phosphate buffered saline with 0.1% bovine serum albumin) with 20 mM sodium citrate (PB/citrate), and centrifuged to pellet cells. The supernatant was reserved, the cells were resuspended in 3 mL of ice cold PB/citrate, then incubated with the magnetic affinity reagent (containing approximately 2 µg of each antibody).

Bound and unbound cells were separated by placing the sample in a Dynal MPC® magnetic particle concentrator, and reserving the unbound material. The bound cells were washed 5-6 times by resuspending in PB (phosphate buffered saline with 0.1% bovine serum albumin), collecting bound cells using the Dynal MPC, and reserving the unbound material.

The reserved supernatant and unbound material was reprocessed to capture any unbound trophoblast cells by adding addition magnetic affinity reagent and processing as above. The bead/cell complexes were combined after washing was completed. The magnetic beads were removed from the isolated cells by incubation in DNase according the manufacturer's instructions, and collected by centrifugation. The collected cells were resuspended in PB/citrate and used to make cell smears.

Cells from the CVS and isolated trophoblast cells were assayed by FISH for presence of Y chromosomes. The samples were double stained with DNA probes for the X and Y chromosomes (CEP X Aqua and CEP Y Orange probes from Vysis, Inc.). CVS cells from all three women were positive for Y chromosomes, indicating a male fetus in each case. One of the three samples of trophoblast cells from the maternal central blood (from 37626) stained with the Y chromosome probe, indicating that fetal trophoblast cells can isolated from a small volume maternal central blood sample after only a single enrichment step.

The patents, patent applications, and publications cited throughout the disclosure are incorporated herein by reference in their entirety.

The present invention has been detailed both by direct description and by example. Equivalents and modifications of the present invention will be apparent to those skilled in the art, and are encompassed within the scope of the invention.

## Claims

1. A method of isolating trophoblast cells from a pregnant female individual, comprising:
obtaining a central maternal blood sample from said individual;
contacting said blood sample with an affinity reagent specific for a trophoblast cell marker; and
isolating trophoblast cells.

2. The method of claim 1, wherein said central maternal blood sample is obtained from said individual's uterine wall.

3. The method of claim 2, wherein said central maternal blood sample is obtained by inserting a needle into the face of said individual's cervix

4. The method of claim 2, wherein said central maternal blood sample is obtained by inserting a needle through said individual's uterine os and into said individual's uterine wall.

5. The method of claim 1, wherein said central maternal blood sample is obtained at from about 6 to about 16 weeks of gestation.

6. The method of claim 1, wherein said central maternal blood sample is obtained using a needle that is at least 20 gauge.

7. The method of claim 6, wherein said needle is at least 18 gauge.

8. The method of claim 1, wherein said trophoblast cells are isolated using an affinity reagent that binds a trophoblast specific marker.

9. The method of claim 1, wherein said trophoblast cells are isolated using an affinity reagent that binds a trophoblast associated marker.

10. The method of claim 1, wherein said trophoblast cells are isolated using a combination of at least two affinity reagents.

11. The method of claim 1, wherein said trophoblast cells are isolated using an antibody.

12. The method of claim 11, wherein said antibody is a monoclonal antibody.

13. The method of claim 11, wherein said antibody binds to a trophoblast specific marker.

14. The method of claim 11, wherein said antibody binds to a trophoblast associated marker.
